(19) Europäisches Patentamt
European Patent Office
Office européen des brevets

(11) Numéro de publication: **0 051 020**
**B1**

# (12) FASCICULE DE BREVET EUROPEEN

(45) Date de publication du fascicule du brevet: **08.08.84**

(51) Int. Cl.³: **C 07 D 453/06**, A 61 K 31/435

(21) Numéro de dépôt: **81401642.4**

(22) Date de dépôt: **20.10.81**

## (54) Acides aza bicyclooctane carboxyliques, leur préparation et compositions pharmaceutiques les contenant.

(30) Priorité: **21.10.80 FR 8022438**

(43) Date de publication de la demande: **05.05.82 Bulletin 82/18**

(45) Mention de la délivrance du brevet: **08.08.84 Bulletin 84/32**

(84) Etats contractants désignés: **AT BE CH DE FR GB IT LI LU NL SE**

(56) Documents cités:
**US - A - 3 833 562**

**CHEMISCHE BERICHTE, vol. 98, no. 5, 1965 (DE) R. ALBRECHT et al.: "Heterocyclen durch Diensynthese. II. *N-(Butyloxycarbonylmethylen)-p-toluosulfonamid, ein neues Dienophil zur Darstellung von Pyridin-. Piperidein- und Piperidin-Derivaten durch Diels-Alder-Synthese*", pages 1431-1434**

(73) Titulaire: **ADIR, 22, rue Garnier, F-92200 Neuilly-sur-Seine (FR)**

(72) Inventeur: **Vincent, Michel, 8 Allée du Prunier Hardy, F-92220 Bagneux (FR)**
Inventeur: **Remond, Georges, 9 av. des Etats-Unis, F-78000 Versailles (FR)**
Inventeur: **Lauble, Michel, 35 av. Foch, F-92420 Vaucresson (FR)**

**EP 0 051 020 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte à des acides azabicyclooctane carboxyliques, à leur préparation et aux compositions pharmaceutiques les contenant.

Plus particulièrement, elle concerne des composés de formule générale (I):

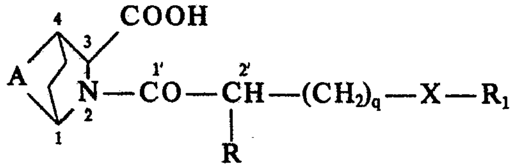

(I)

dans laquelle A représente un radical vinylène ou diméthylène

q est 0 ou 1,
R représente un radical alkyle inférieur pouvant porter un groupe amino
X représente —S— et $R_1$ représente H, ou bien X représente —NH— et
$R_1$ représente un atome d'hydrogène ou un radical de formule

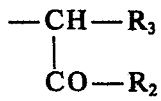

$R_2$ représente un hydroxyle ou un groupement alcoxy inférieur
$R_3$ représente un atome d'hydrogène, un radical alkyle linéaire ou ramifié, cycloalkyle ou phénylalkyle, ayant au plus et au total 8 atomes de carbone, ou un radical de formule:

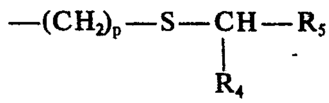

dans lequel:

$R_4$ est H, un radical alkyle inférieur ou cycloalkyle ($C_3$ à $C_6$),
$R_5$ est H, un radical cycloalkyle ($C_3$ à $C_6$) ou alcoxy (inférieur)-carbonyle, et
p est 1 ou 2.

On entend par radicaux alkyle ou alcoxy inférieurs des groupes ayant de 1 à 4 atomes de carbone.

On connait le brevet des Etats Unis N° 3 833 562 concernant des azabicycloalcanes oxygénés pouvant porter des substituants aralkyle ou acyle sur l'atome d'azote du cycle, composés qui sont actifs sur le S.N.C. et comme agents fongicides. Or, les composés selon la présente invention sont des dérivés d'alpha-aminoacides, portant un groupe carboxy en position alpha de l'azote du cycle, et éventuellement un second groupe alpha-aminoacide sur la chaîne latérale, chacun de ces atomes dàzote étant engagé dans une liaison peptidique. Cette structure particulière des composés leur procure une activité sur le système de conversion de l'angiotensine responsable de certains cas d'hypertension.

L'invention se rapporte également aux sels de composés de formule générale (I) obtenus avec une base minérale ou organique thérapeutiquement compatible.

L'invention se rapporte également aux sels d'addition des composés de formule (I) dans laquelle X est NH avec un acide minéral ou organique thérapeutiquement compatible.

Les composés de formule (I) comportent au moins 2 atomes de carbone asymétrique. Selon la position des substituants et le degré d'hydrogénation, il existe 2 à 8 centres d'asymétrie.

Les composés racémiques peuvent être dédoublés en leurs mélanges de diastéréoisomères ou d'épimères, ou dédoublés en leurs énantiomères de manière connue. Ces divers isomères font partie de l'invention de même que les composés racémiques.

L'invention comprend plus particulièrement les composés de formule générale (I) dans laquelle X représente NH et $R_3$ représente un groupe alkyle ou phénylalkyle ayant au plus 8 atomes de carbone. De plus, les composés de formule (I) dans laquelle A représente un groupe diméthylène sont préférés et R représente avantageusement un groupe méthyle.

Les composés selon l'invention ainsi que leurs sels sont doués de propriétés pharmacologiques intéressantes. Ils inhibent notamment la transformation du décapeptide angiotensine I en l'octapeptide angiotensine II par une inhibition de l'enzyme de conversion.

Les composés selon l'invention ont une action inhibitrice sur les enzymes comme les carboxypolypeptidases ou les enképhalinases. Leur emploi en thérapeutique permet donc de réduire ou même de

supprimer l'action de ces enzymes, en agissant sur un des mécanismes directement responsables de l'hypertension ou de l'insuffisance cardiaque.

L'invention se rapporte donc à l'emploi en thérapeutique des composés de formule générale (I) et de leurs sels, notamment pour le traitement de l'hypertension artérielle et de l'insuffisance cardiaque.

L'invention s'étend aussi aux compositions pharmaceutiques renfermant comme principe actif au moins un composé de formule générale I ou un de ses sels d'addition, avec un excipient inerte, non toxique, pharmaceutiquement acceptable.

En vue de l'emploi en thérapeutique, les composés de formule générale (I) on leurs sels sont présentés sous des formes pharmaceutiques convenant pour l'administration par voie intraveineuse ou orale. Les compositions pharmaceutiques selon l'invention renferment, outre le principe actif, un ou plusieurs axcipients inertes, nontoxiques convenant pour l'usage pharmaceutique et/ou un agent liant, un agent aromatisant, un agent de délitement, un agent édulcorant, un agent lubrifiant ou bien encore un véhicule liquide adapté à l'administration par voie intraveineuse.

Les compositions pharmaceutiques selon l'invention peuvent en outre contenir un autre principe actif d'action synergique ou complémentaire. Parmi ces derniers principes actifs, on pourra citer un diurétique et, notamment, un saliurétique, comme par exemple un thiazide, un dihydrothiazide, un chloro sulfamide, un acide dihydrobenzofuran 2-carboxylique ou un dérivé de l'acide phénoxy acétique. Des exemples de tels composés sont la N (3'-chloro 4'-sulfamyl benzamido) 2-méthyl indoline, l'acide éthacrynique, le furosémide.

On pourra également ajouter une substance $\alpha$-adrénolytique, un $\beta$-bloqueur, un antagoniste calcique ou un agoniste des récepteurs dopaminergiques vasculaires.

La posologie utile peut varier largement en fonction de l'âge, du poids du patient, de la sévérité de l'indication thérapeutique ainsi que la voie d'administration. La voie d'administration préférée est la voie orale mais la voie intraveineuse est également parfaitement appropriée au traitement de l'hypertension.

D'une manière générale, la posologie unitaire s'échelonnera entre 10 et 200 mg.

L'invention comprend également un procédé d'obtention des composés de formule générale (I), selon lequel on soumet un acide azabicyclooctane carboxylique ou l'un de ses esters, de formule générale (II)

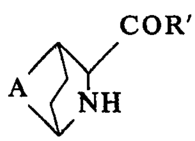

(II)

dans laquelle la définition du substituant A est la même que dans la formule (I), et R' représente un hydroxy ou alcoxy inférieur, à l'action d'un acide carboxylique substitué de formule générale (III):

$$X'-(CH_2)_q-\underset{\substack{|\\R''}}{CH}-COOH \qquad \text{(III)}$$

dans laquelle q a la définition mentionnée pour la formule (I), et R'' est un alkyle inférieur ou aminoalkyle protégé, X' représente SH ou un groupement $NH_2$ protégés par les radicaux acyles habituels tels que par exemple acétyle, benzyloxy carbonyle ou terbutoxycarbonyle, ou un de ses dérivés fonctionnels.

Pour obtenir un dérivé d'acide de formule générale (IV):

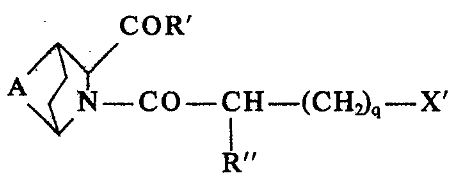

(IV)

dans laquelle les substituants ont les définitions déjà mentionnées ci-dessus, qui est soumis aux procédés de déprotection habituel tels que saponification et/ou hydrogénolyse et fournit ainsi un composé de formule (I) dans laquelle $R_1$ est H, puis le cas échéant, on soumet le composé de formule (I) dans laquelle $R_1$ est H, et X est NH à une réaction d'alkylation réductive par un composé de formule générale (V)

$$R_2-CO-CO-R_3 \qquad \text{(V)}$$

dans laquelle $R_2$ et $R_3$ ont les significations indiquées dans la formule (I) pour obtenir un composé de

formule (I) dans laquelle X est NH et $R_1$ est

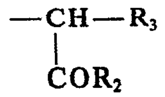

Les composés intermédiaires de formule générale (II) ont été préparés par un procédé selon lequel on soumet une imidazo pyridine, de formule générale (VI):

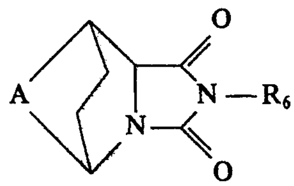

(VI)

dans laquelle A a la signification mentionnée ci-dessus et $R_6$ est un radical tel que phényle substitué ou non, à une réaction d'hydrolyse par l'un des procédés habituels tel que l'ébullition avec une solution aqueuse de soude. Les composés de formule (VI) sont décrits par BEN-ISHAI et GOLDSTEIN (Tétrahedron, 27, pp. 3119—3127 [1971]).

Les exemples suivants illustrent l'invention.

## Exemple 1

### N [mercapto-3 (RS) méthyl-2 propionyl] aza-2 (RS) carboxy-3 bicyclo [2,2,2] octane

### Stade A

### Aza-2 (RS) carboxy-3 bicyclo [2,2,2] octane

3,8 g (0,0148 mol) de dihydro-8,8 a dioxo-1,3 éthano-5,8 phényl-2 (2H, 5H) imidazo [1,5-a] pyridine (préparé selon D. BEN-ISHAI et E. GOLDSTEIN, Tétrahédron, vol. 27, p. 3119—3127) sont mis en suspension dans une solution de 30 ml de soude 4 N et de 10 ml de méthanol. Le mélange est porté au reflux pendant 24 heures, refroidi, filtré et le filtrat acidifié par 30 ml d'acide chlorhydrique 4 N puis passé sur 200 ml de résine échangeuse d'ion Dowex 50 $H^+$.

Après lavage de la résine à l'eau distillée jusqu'à absence d'ion chlorure dans l'éluat, le composé cherché est élué par 500 ml d'ammoniaque N/1. Les éluats ammoniacaux évaporés à sec laissant un résidu qui est le produit cherché.

Poids: 1,8 g (78%)
Point de fusion (Bloc Kofler): 253—255°

### Stade B

### N [Acétylthio-3 (RS) méthyl-2 propionyl] aza-2 (RS) carboxy-3 bicyclo [2,2,2] octane

0,850 g (0,0055 mol) d'Aza-2 (RS) carboxy-3 bicyclo [2,2,2] octane obtenu au stade précédent sont mis en suspension dans une solution de 1,33 g (0,011 mol) de N diméthyl aniline dans 40 ml de chlorure de méthylène.

1 g (0,0055 mol) de chlorure de l'acide acéthylthio-3 (RS) méthyl-2 propionique sont ajoutés goutte à goutte en 5 minutes à la solution précédente agitée à température ambiante. L'agitation est poursuivie pendant 15 heures. La solution obtenue est coulée sur un mélange comprenant 150 g de glace pilée et 30 ml de solution aqueuse normale d'acide chlorhydrique. La phase organique est soutirée, lavée à HCl N puis à l'eau distillée jusqu'à neutralité, séchée sur $SO_4$ Ca, filtrée concentrée à sec et le résidu d'évaporation chromatographié sur silice Merck F 254 en éluant par un mélange chlorure de méthylène-méthanol (95/5).

0,550 g (34%) de produit cherché est obtenu sous forme d'une huile dont les spectres IR et RMN sont conformes à la structure attendue.

Stade C

N [mercapto-3 (RS) méthyl-2 propionyl] aza-2 (RS) carboxy-3 bicyclo [2,2,2] octane

Sous azote, 0,500 g (0,0017 mol) de N [acétylthio-3 (RS) méthyl-2 propionyl] aza-2 (RS) carboxy-3 bicyclo [2,2,2] octane préparés au stade précédent sont dissous dans un mélange de 1,7 ml de soude aqueuse normale et de 25 ml d'éthanol. Après 15 heures de contact l'éthanol est évaporé sous vide et la solution aqueuse extraite à l'éther, neutralisée exactement par 1,7 ml d'acide chlorhydrique aqueux normale est évaporé à sec. Le résidu constitue le produit cherché (0,3 g, en mélange avec 0,0995 g de chlorure de sodium. Le produit est contrôlé par RMN en solution dans $D_2O$:

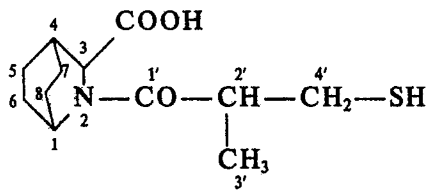

| | |
|---|---|
| 2H en 1 et 3 | $\delta$=4,10 et 4,40 ppm |
| 3H en 2′ et 4′ | $\delta$=2,3 à 3 ppm |
| 1H en 4 | $\delta$=2 à 3 ppm (multiplet) |
| 8H (4 $CH_2$) en 5,6,7,8) | $\delta$=1,7 ppm (massif) |
| 3H en 3′ | $\delta$=1,5 ppm (doublet) |

l'intégration est cohérente.

Exemple 2

N [N-(RS) éthoxycarbonyl-1 pentyl (3) alanyl] aza-2 (RS) carboxy-3 bicyclo (2,2,2) 4 (RS), 7 (RS) octène-5

Stade A

Aza-2 (RS) carboxy-3 bicyclo (2,2,2) 4 (RS), 7 (RS) octène-5

34,5 g (0,119 mole) de dioxo-1,3 ethano-5,8 (chloro-4-phényl)-2 (2H, 5H) imidazo [1,5-a] pyridine préparé selon BEN-ISHAI et coll. (voir. Ex. 1,A) sont portés à reflux sous azote pendant 5 h. avec 355 ml (1,42 mole) de solution aqueuse de soude 4N.

Après refroidissement à 5°, 14 g de chloro-4 aniline sont essorés et le filtrat est acidifié à PH 1 par HCl concentré. La solution filtrée est passée sur 800 ml de résine (Dowex ($H^+$) 50 WX-8). Après lavage par de l'eau distillée jusqu'à absence d'ion chlorure, le produit cherché est élué par 2250 ml d'ammoniaque 1N. Les éluats ammoniacaux sont concentrés à sec sous vide de la trompe à eau à 40°. Poids 17,5 g (Rt=96,2%).

Analyse: $C_8H_{11}NO_2$

| | | | |
|---|---|---|---|
| Calculé % | C 62,72, | H 7,24 | N 9,14; |
| Trouvé % | C 62,30, | H 6,87, | N 9,10. |

| | | |
|---|---|---|
| IR: | OH et $NH_2^+$ | 3600—3200 $cm^{-1}$ |
| | $COO^-$ | 1630 $cm^{-1}$ |

RMN: ($D_2O$-intégration cohérente)

| | | | |
|---|---|---|---|
| 4H | 1,3—2,1 ppm | | |
| 2H | 6,5 ppm | 1H | 4,3 ppm |
| 1H | 3,7 ppm | 1H | 3,25 ppm |

Stade B

Aza-2 (RS) méthoxycarbonyl-3 bicyclo (2,2,2) 4 (RS), 7 (RS) octène-5, chlorhydrate

1 g (0,00655 mole) d'aminoacide préparé au stade précédent sont dissous dans 15 ml de méthanol anhydre et additionnés, goutte à goutte et sans dépasser ±5°, de 1,5 mole de chlorure de thionyle. Le

mélange est porté au reflux pendant 2 h puis concentré à sec sous vide de la trompe à eau à 40°. On obtient 1,2 g (Rt. 90%) de produit cherché.

F: 207 (déc.)
IR: CO (ester) 1740 $cm^{-1}$
$NH_2^+$ 2800—2200 $cm^{-1}$

Le produit brut est utilisé dans la phase suivante sans purification supplémentaire.

Stade C

N [N(t-butoxycarbonyl)alanyl] aza-2 (RS) méthoxycarbonyl-3 bicyclo [2,2,2] 4 (RS), 7 (RS) octène-5

8,9 g (0,044 mole) d'ester prépare selon le procédé décrit au stade précédent sont dissous dans 70 ml de diméthylformamide (DMF) en présence de 6,15 ml (0,044 mole) de triéthylamine. A la solution obtenue, maintenue à température ambiante, on ajoute successivement:

— 8,3 g de (S) ter. boc. alanine dissoute dans 45 ml de DMF
— 6,45 (0,044 mole) d'hydroxy-benztriazole (HOBT) dissous dans 55 ml de DMF, et
— 9,05 (0,044 mole) de dicyclohexylcarbodiimide (DCCI) dissous dans 80 ml de chloroforme.

Après 24 h. d'agitation, la dicyclohexylurée (DCU) formée est filtrée et le filtrat concentré à sec sous vide de la trompe à eau à 50°C. Le résidu est repris par 250 ml d'acétate d'éthyle, et la solution est filtrée et lavée successivement par:

2 x 50 ml de solution aqueuse saturée de NaCl
3 x 50 ml de solution aqueuse d'acide citrique à 10%
2 x 50 ml de solution aqueuse saturée de NaCl
3 x 50 ml de solution aqueuse saturée de $NaHCO_3$
2 x 50 ml de solution aqueuse saturée de NaCl,

puis séchée sur $CaSO_4$, filtrée et concentré à sec.
On obtient 12,8 g (86,5%) de produit cherché sous forme d'une huile très visqueuse.

Analyse: $C_{17}H_{26}N_2O_5$

| | | | |
|---|---|---|---|
| Calculé % | C 60,34, | H 7,74, | N 8,28; |
| Trouvé % | C 60,10, | H 7,79, | N 8,21. |

IR: NH 3400—3300 $cm^{-1}$
CO amide 1700 $cm^{-1}$ et 1510 $cm^{-1}$
CO ester 1750 $cm^{-1}$

RMN: 2H (6,1—6,8 ppm)
1H (5,2—5,8 ppm) échangeable
3H (4,2—4,9 ppm)
3H (3,73 ppm)
1H )3,2 ppm)
9H (1,5 ppm)
7H (1,2—2,2 ppm)

Stade D

N [N-(t-butoxycarbonyl) (S) alanyl] aza-2 (RS) carboxy-3 bicyclo (2,2,2) 4 (RS), 7 (RS) octène-5

12,8 g (0,038 mole) de composé obtenu au stade précédent sont dissous dans 140 ml de méthanol en présence de 40 ml de soude 1N. Après 8 h. d'agitation à température ambiante, la solution est concentrée à sec sous vide de la trompe à eau à 30° et le résidu, redissous dans 150 ml d'eau, est extrait par un peu d'acétate d'éthyle pour séparer les insaponifiables,puis acidifié par 40 ml de HCl 1N. L'acide précipité est extrait par 2 x 100 ml d'éther sulfurique, la solution éthérée est séchée sur $CaSO_4$, filtrée

et concentrée à sec. On obtient 11,1 g (Rt 90%) de produit cherché.

IR: NH et OH 3420 $cm^{-1}$ et 3300—2300 $cm^{-1}$
CO (acide et amide) 1700 $cm^{-1}$
CO (amide tertiaire) 1635 $cm^{-1}$ et 1500 $cm^{-1}$

RMN: 1H (8,4 ppm) échangeable
2H (6,3—6,6 ppm)
1H (5,5 ppm) échangeable
3H (3,9—4,8 ppm)
1H (2,9—3,5 ppm)
16H (1—2 ppm)

### Stade E

N [(S) alanyl] aza-2 (RS) carboxy-3 bicyclo (2,2,2) 4 (RS), 7 (RS) octène-5; (composé n° 2 du tableau suivant)

11,1 g (0,034 mole) de composé obtenu au stade précédent sont dissous dans 95 ml de chlorure de méthylène et à cette solution, refroidie à 0, +5°, sous agitation, sont ajoutés goutte à goutte 75 ml d'acide trifluoroacétique en solution dans 80 ml de chlorure de méthylène. Après 1 h de contact sous agitation à 0, +5°, puis 1 h supplémentaire à +25°, la solution est concentrée à sec sous vide de la trompe à eau puis de la pompe à palette (0,1 mm de Hg).

Le résidu brut (13,6 g) obtenu, est passé en solution aqueuse sur résine (DOWEX 50 $H^+$), la résine est lavée à l'eau distillée puis le produit cherché est élué par 1 l d'ammoniaque 1N. L'évaporation à sec des éluats ammoniacaux fournit le produit attendu.

Poids 6,3 g (Rt=83%)

Analyse: $C_{11}H_{16}N_2O_3$

| | | | |
|---|---|---|---|
| Calculé % | C 58,91, | H 7,19, | N 12,50; |
| Trouvé % | C 57,80, | H 7,09, | N 12,83. |

IR et RMN: se reporter au tableau.

### Stade F

N [N-(RS) éthoxycarbonyl-1 pentyl (S) alanyl] aza-2 (RS) carboxy-3 bicyclo (2,2,2) 4 (RS), 7 (RS) octène-5;
(composé N° 8 du tableau suivant)

1 g (0,045 mole) de composé obtenu au stade précédent est dissous sous agitation dans 55 ml d'éthanol anhydre en présence de 13 g de tamis moléculaire 4 Å et de 2,85 g (0,018 mole) d'oxo-2 hexanoate d'éthyle ($E_{15}$=89—91°C, préparé selon P. A. MANIS et M. W. RATHKE, J. Org. Chem., 45, 4952—54 [1980]). Après 1 h. d'agitation à température ambiante, une solution de 0,28 g (0,0045 mole) de cyano borohydrure de sodium dans 2,25 ml d'éthanol anhydre est ajoutée en 6 h. L'agitation est continuée pendant 15 h., puis la solution filtrée et concentrée à sec est reprise par 50 ml de solution aqueuse de NaCl. Après extraction à l'éther pour séparer l'excès de céto-ester, la phase aqueuse est amenée à pH 3 par un peu de HCl 1N puis extraite à l'acétate d'éthyle. La phase organique est séchée sur $CaSO_4$ puis filtrée et concentrée à sec. Le résidu d'évaporation est le produit cherché sous forme de sel de sodium.

Analyse: $C_{19}H_{29}N_2NaO_5$

| | | | |
|---|---|---|---|
| Calculé % | C 58,75, | N 7,53, | N 7,21; |
| Trouvé % | C 58,74, | H 7,71, | N 7,48. |

Dans le tableau suivant sont rassemblés les composés des exemples ci-dessus ainsi que d'autres composés de formule (I) préparés de la même manière.

| N° composé | A (chiralité du $C_3$) | q | X | R (chiralité du $C_{2'}$) | $R_1$ | forme |
|---|---|---|---|---|---|---|
| 1 (ex. 1) | $CH_2$—$CH_2$ (RS) | 1 | S | —$CH_3$ (RS) | H | – |
| 2 (ex. 2–E) | CH═CH (RS) | 0 | NH | $CH_3$ (S) | H | – |
| 3 | $CH_2$—$CH_2$ (RS) | 0 | NH | $CH_3$ (S) | H | – |
| 4 | $CH_2$—$CH_2$ (RS) | 0 | NH | $CH_3$ (S) | (RS) —$CH(COOC_2H_5)CH_2$—S— 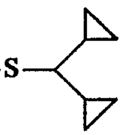 | – |
| 5 | $CH_2$—$CH_2$ (RS) | 0 | NH | $CH_3$ (S) | (RS) —$CH(COOC_2H_5)CH_2$—$CH(CH_3)_2$ | sel sodique |
| 6 | $CH_2$—$CH_2$ (R) | 0 | NH | $CH_3$ (S) | (RS) —$CH(COOC_2H_5)CH_2CH(CH_3)_2$ | sel sodique |
| 7 | $CH_2$—$CH_2$ (S) | 0 | NH | $CH_3$ (S) | (RS) —$CH(COOC_2H_5)CH_2CH(CH_3)_2$ | sel sodique |
| 8 (ex. 2) | CH═CH (RS) | 0 | NH | $CH_3$ (S) | (RS) —$CH(COOC_2H_5)$—n $C_4H_9$ | sel sodique |
| 9 | $CH_2$—$CH_2$ (RS) | 0 | NH | —$CH_3$ (S) | (RS) —$CH(COOC_2H_5)$—n $C_4H_9$ | sel sodique |
| 10 | $CH_2$—$CH_2$ (S) | 0 | NH | —$CH_3$ (S) | (RS) —$CH(COOC_2H_5)$—n $C_4H_9$ | sel sodique |
| 11 | $CH_2$—$CH_2$ (R) | 0 | NH | —$CH_3$ (S) | (RS) —$CH(COOC_2H_5)$—n $C_3H_7$ | sel sodique |
| 12 | $CH_2$—$CH_2$ (S) | 0 | NH | —$CH_3$ (S) | (RS) —$CH(COOC_2H_5)$—n $C_3H_7$ | sel sodique |

Fortsetzung

| N° composé | A (chiralité du $C_3$) | q | X | R (chiralité du $C_{2'}$) | $R_1$ | forme |
|---|---|---|---|---|---|---|
| 13 | $CH_2—CH_2$ (S) | 0 | NH | $—CH_3$ (S) | (RS) $—CH(COOC_2H_5)—n\,C_5H_{11}$ | – |
| 14 | $CH_2—CH_2$ (S) | 0 | NH | $—CH_3$ (S) | 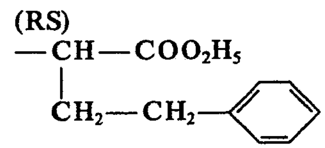 | sel sodique |
| 15 | $CH_2—CH_2$ (S) | 0 | NH | $—CH_3$ (S) | 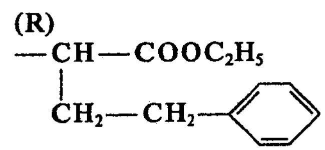 | trifluoro-acétate |
| 16 | $CH_2—CH_2$ (S) | 0 | NH | $—CH_3$ (S) | 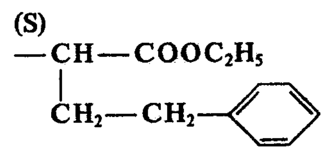 | trifluoro-acétate |
| 17 | $CH_2—CH_2$ (S) | 0 | NH | $CH_3$ (S) | 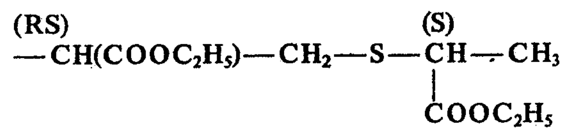 | sel sodique |

| IR: $\nu_S$ en $cm^{-1}$ | | RMN dans CDCl3: déplacements chimiques ppm/TMS | | | N° composé |
|---|---|---|---|---|---|
| — | | se reporter à l'exemple 1 stade C | | | 1 |
| NH, $NH_3^+$, OH:<br>C=O: | 3600—2200<br>1650—1550 | 2H (6,5—6,2)<br>1H (5,1) | 2H (4,5—3,5)<br>1H (3) | 7H (2—1,1)<br>RMN dans $D_2O$ | 2 |
| NH, OH:<br>C=O: | 3600—2400<br>1650—1550 | 3H (4,5—3,7)<br>1H (2,15) | 8H (1,65)<br>3H (1,4—1,1) | RMN dans $D_2O$ | 3 |
| $NH_2^+$:<br>C=O ester:<br>C=O amide: | 3500—2500<br>1725<br>1625 | 2H (5,8) échangeables<br>8H (4,5—2,8) | 26H (2,3—0,1) | | 4 |
| NH:<br>C=O ester:<br>C=O amide: | 3300<br>1720<br>1650—1580 | 7H (4,5—3)<br>24H (2,5—0,7) | | | 5 |
| NH, OH:<br>C=O ester:<br>C=O amide: | 3320<br>1725<br>1610 | 1H (4,8) échangeable<br>6H (2,5—0,5) | 24H (1) | | 6 |
| NH, OH:<br>C=O ester:<br>C=O amide: | 3320<br>1725<br>1610 | 1H (4,5—3)<br>échangeable<br>6H (2,5—0,7) | 24H (0,95) | | 7 |
| $NH_2^+$, OH:<br>C=O ester:<br>C=O amide: | 3600—2300<br>1735<br>1640 | 1H (8) échangeables<br>2H (6,5) | 6H (4,7—3,1)<br>20H (2,4—0,8) | | 8 |
| $NH_2^+$:<br>OH:<br>C=O ester:<br>C=O amide: | 2700—2300<br>3600—3200<br>1730<br>1630 et 1540 | 2H (7,5) échangeables<br>6H (4,7—3,5) | 24H (2,5—0,6) | | 9 |
| NH, OH:<br>C=O amide:<br>C=O ester: | 3400<br>1610<br>1725 | 7H (4,6—2,9)<br>24H (2,5—0,7) | | | 10 |
| NH, OH:<br>C=O ester:<br>C=O amide: | 3600—3100<br>1725<br>1620 | 7H (4,5—3)<br>22H (2,6—0,8) | | | 11 |
| NH, OH:<br>C=O ester:<br>C=O amide: | 3600—3100<br>1725<br>1620 | 7H (4,7—3)<br>22H (2,6—0,3) | | | 12 |
| NH, OH:<br>C=O ester:<br>C=O amide: | 3600—2300<br>1725<br>1630 | 2H (6,2) échangeable<br>6H (4,5—2,5) | 26H (2,5—0,6) | | 13 |
| NH, OH:<br>C=O ester:<br>C=O amide: | 3300<br>1725<br>1615 | 5H (7,3)<br>6H (4,5—3) | 19H (3—1) | | 14 |
| NH, OH:<br>C=O ester:<br>C=O amide: | 2800—2300<br>1730<br>1670 et 1630 | | | | 15 |
| $NH_2^+$, OH:<br>C=O ester:<br>C=O amide: | 3200—2200<br>1730<br>1620 | 3H (8,35) échangeables<br>5H (7,35) | 6H (4,8—3,7)<br>4H (3—2,2) | 12H (2,2—1,6)<br>3H (1,4) | 16 |
| NH, OH:<br>C=O ester:<br>C=O amide: | 3400<br>1725<br>1615 | 11H (4,6—2,8)<br>22H (2—1) | | | 17 |

Etude pharmacologique des composés de l'invention.

Les composés selon l'invention ont été testés par l'administration i.v. ou p.o. sur le chien éveillé.

La pression artérielle des chiens a été mesurée par un capteur de pression (»Statham P 23 Db«) après cathétérisation de l'aorte par l'intermédiaire de l'artère fémorale. L'enregistrement est réalisé par un appareil enregistreur (»Brush 400«).

L'angiotensine I et l'angiotensine II sont injectées aux animaux par voie intra-veineuse à la dose de 0,3 γ/kg. On établit une courbe dose/activité pour chacune de ces hormones. On administre ensuite les composés selon l'invention par voie orale ou intra-veineuse à la dose de 1 à 10 mg/kg. On établit ensuite une deuxième courbe dose/activité pour l'angiotensine I et pour l'angiotensine II après administration du produit essayé.

On constate une inhibition de l'activité hypertensive allant de 50 à 100% après 30 à 90 minutes et se maintenant de 40 à 80% à plus de 6 h. après l'administration. Certains composés restent actifs après 24 h., ce qui n'est le cas d'aucun composé de ce type connu jusqu'à présent. Par ailleurs, les composés de l'invention sont dépourvus de toxicité aigüe ($DL_0 > 500$ mg/kg i;p; souris).

Exemple de Formulation

N [mercapto-3 (RS) méthyl-2 propionyl] aza-2 (RS) carboxy-3 bicyclo [2,2,2]

| | |
|---|---|
| octane | 20 g |
| amidon de blé | 105 g |
| amidon de maïs | 90 g |
| caséine formolé | 20 g |
| stéarate de magnésium | 15 g |
| talc | 20 g |

pour 100 comprimés.

**Revendications pour les Etats contractants: BE, CH, DE, FR, GB, IT, LI, LU, NL, SE**

1. Composés répondant à la formule générale:

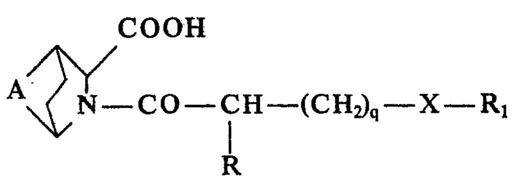

(I)

dans laquelle A représente un radical vinylène ou diméthylène

q est 0 ou 1
R représente un radical alkyle de $C_1$ à $C_4$ pouvant porter un groupe amino,
X représente —S— et $R_1$ représente H, ou bien X représente —NH— et
$R_1$ représente un atome d'hydrogène ou un radical de formule

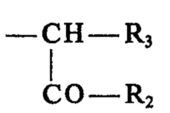

$R_2$ représente un hydroxyle ou un groupement alcoxy de $C_1$ à $C_4$,
$R_3$ représente un atome d'hydrogène, un radical alkyle droit ou ramifié, cycloalkylalkyle ou phénylalkyle ayant au plus et au total 8 atomes de carbone, ou un radical de formule:

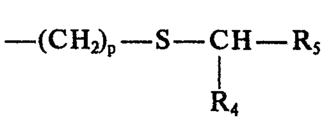

dans lequel:

$R_4$ est H, un radical alkyle ($C_1$ à $C_4$) ou cycloalkyle ($C_3$ à $C_6$),
$R_5$ est H, un radical cycloalkyle ($C_3$ à $C_6$) ou alcoxy ($C_1$ à $C_4$) carbonyle, et
p est 1 ou 2,

sous forme racémique ou d'isomère optique, ainsi que leurs sels obtenus avec une base minérale ou organique thérapeutiquement compatible et les sels d'addition des composés de formule (I) dans laquelle X est NH avec un acide minéral ou organique thérapeutiquement compatible.

2. Composés selon la revendication 1, répondant à la formule (I) dans laquelle: A représente un groupe diméthylène.

3. Composés selon la revendication 1, ou la revendication 2, répondant à la formule (I) dans laquelle X représente NH et $R_3$ représente un groupe alkyle ou phénylalkyle ayant au plus 8 atomes de carbone.

4. Composés selon l'une quelconque des revendications 1 à 3, répondant à la formule (I) dans laquelle R est un groupe méthyle.

5. Le N [thio-3 (RS) méthyl-2 propionyl] aza-2 (RS) carboxy-3 bicyclo [2,2,2] octane et ses isomères (S).

6. Le N [N-3 (RS) (éthoxycarbonyl-1 méthyl-3 butyl) (S) alanyl] aza-2 (S) carboxy-3 bicyclo (2,2,2) octane, son isomère (S) et leur sel de sodium.

7. Le N [N-(RS) (éthoxycarbonyl-1 phényl-3 propyl) (S) alanyl] aza-2 (S) carboxy-3 bicyclo (2,2,2) octane, son isomère (S), leur sel de sodium et leur trifluoroacétate.

8. Composition pharmaceutique renfermant à titre de principe actif au moins uncomposé selon l'une quelconque des revendications 1 à 7, ainsi qu'un excipient ou un véhicule inerte non-toxique convenable.

9. Procédé de préparation de composés selon la revendication 1, procédé caractérisé en ce que l'on soumet un acide aza bicyclooctane carboxylique ou l'un de ses esters de formule générale (II):

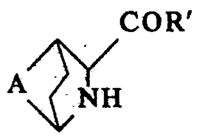

(II)

dans laquelle la définition du substituant A est la même que dans la formule (I) de la revendication 1, et R' représente un hydroxy ou alcoxy de $C_1$ à $C_4$, à l'action d'un acide carboxylique substitué de formule générale (III):

$$X'-(CH_2)_q-\underset{\displaystyle R''}{\underset{|}{CH}}-COOH \qquad (III)$$

dans laquelle q a la définition mentionnée pour la formule (I) de la revendication 1, et R'' est alkyle ($C_1$ à $C_4$) ou aminoalkyle ($C_1$ à $C_4$) protégé, X' représente SH ou un groupement $NH_2$ protégés par les radicaux acyles habituels, ou un de ses dérivés fonctionnels, pour obtenir un acide de formule générale (IV):

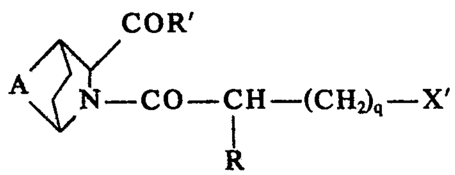

(IV)

dans laquelle les substituants ont les définitions déjà mentionnées ci-dessus, qui est soumis aux procédé de déprotection habituels, et fournit ainsi le composé de formule générale (I) dans laquelle $R_1$ est H,
puis le cas échéant, on soumet le composé de formule (I) dans laquelle $R_1$ est H et X est NH à une réaction d'alkylation réductive par un composé de formule générale (V)

$$R_2-CO-CO-R_3 \qquad (V)$$

dans laquelle $R_2$ et $R_3$ ont les significations indiquées dans la formule (I), pour obtenir un composé de formule (I) dans laquelle X est NH et $R_1$ est

$$-\underset{\displaystyle COR_2}{\underset{|}{CH}}-R_3$$

**Revendication pour l'État contractant: AT**

Procédé de préparation de composés répondant à la formule générale:

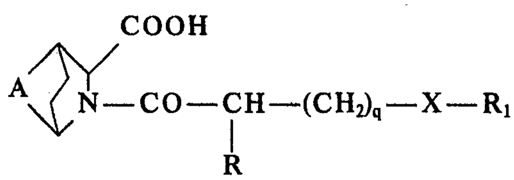

(I)

dans laquelle A représente un radical vinylène ou diméthylène

q est 0 ou 1
R représente un radical alkyle ($C_1$ à $C_4$) pouvant porter un groupe amino,
X représente —S— et $R_1$ représente H, ou bien X représente —NH— et
$R_1$ représente un atome d'hydrogène ou un radical de formule

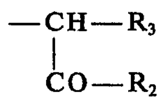

$R_2$ représente un hydroxyle ou un groupement alcoxy ($C_1$ à $C_4$)
$R_3$ représente un atome d'hydrogène, un radical alkyle droit ou ramifié, cycloalkylalkyle ou phénylalkyle ayant au plus et au total 8 atomes de carbone, ou un radical de formule:

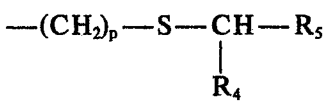

dans lequel:

$R_4$ est H, un radical alkyle ($C_1$ à $C_4$) ou cycloalkyle ($C_3$ à $C_6$),
$R_5$ est H, un radical cycloalkyle ($C_3$ à $C_6$) ou alcoxy ($C_1$ à $C_4$) carbonyle, et
p est 1 ou 2,

sous forme racémique ou d'isomère optique, ainsi que leurs sels obtenus avec une base minérale ou organique thérapeutiquement compatible et les sels d'addition des composés de formule (I) dans laquelle X est NH avec un acide minéral ou organique thérapeutiquement compatible, procédé caractérisé en ce que l'on soumet un acide aza bicyclo-octane carboxylique ou l'un de ses esters de formule générale (II):

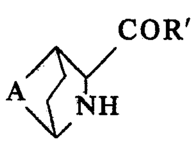

(II)

dans laquelle la définition du substituant A est la même que dans la formule (I), et R' représente un hydroxy ou alcoxy de $C_1$ à $C_4$, à l'action d'un acide carboxylique substitué de formule générale (III):

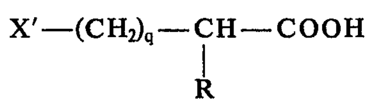

(III)

dans laquelle R et q ont les définitions mentionnées pour la formule (I), et X' représente SH ou un groupement $NH_2$ protégés par les radicaux acyles habituels, ou un de ses dérivés fonctionnels, pour obtenir un acide de formule générale (IV):

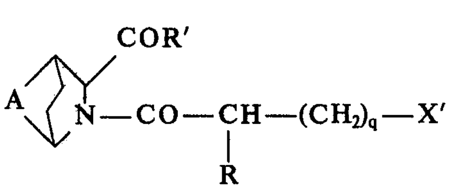

(IV)

dans laquelle les substituants ont les définitions déjà mentionnées ci-dessus, qui est soumis aux procédés de déprotection habituels,et fournit ainsi le composé de formule générale (I) dans laquelle $R_1$ est H, puis le cas échéant, on soumet le composé de formule (I) dans laquelle $R_1$ est H et X est NH à une réaction d'alkylation réductive par un composé de formule générale (V)

$$R_2-CO-CO-R_3 \qquad (V)$$

dans laquelle $R_2$ et $R_3$ ont les significations indiquées dans la formule (I), pour obtenir un composé de formule (I) dans laquelle X est NH et $R_1$ est

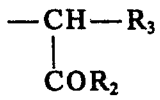

**Patentansprüche für die Vertragsstaaten BE, CH, DE, FR, GB, IT, LI, LU, NL, SE**

1. Verbindungen der allgemeinen Formel

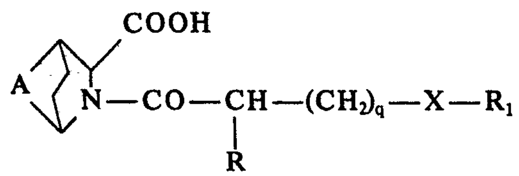

(I)

in der A eine Vinylengruppe oder eine Dimethylengruppe,

q 0 oder 1,
R eine $C_1-C_4$-Alkylgruppe, die eine Aminogruppe tragen kann,
X —S— und $R_1$ H oder X —NH— und $R_1$ ein Wasserstoffatom oder eine Gruppe der Formel

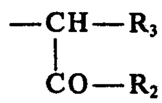

in der

$R_2$ eine Hydroxylgruppe oder eine $C_1-C_4$-Alkoxygruppe und
$R_3$ ein Wasserstoffatom, eine geradkettige oder verzweigte Alkylgruppe, eine Cycloalkylgruppe oder eine Phenylalkylgruppe mit höchstens 8 Kohlenstoffatomen darstellen, oder eine Gruppe der Formel

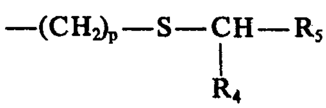

in der

$R_4$ ein Wasserstoffatom, eine $C_1-C_4$-Alkylgruppe oder eine $C_3-C_6$-Cycloalkylgruppe,
$R_5$ ein Wasserstoffatom, eine $C_3-C_6$-Cycloalkylgruppe oder $C_1-C_4$-Alkoxy-carbonylgruppe und
p 1 oder 2 darstellen,

bedeuten, in Form der Racemate oder der optischen Isomeren, sowie deren Salze mit therapeutisch verträglichen anorganischen oder organischen Basen und die Additionssalze der Verbindungen der Formel (I), in der X —NH— bedeutet, mit einer therapeutisch verträglichen anorganischen oder organischen Säure.

2. Verbindungen nach Anspruch 1 der allgemeinen Formel (I), in der A für eine Dimethylengruppe steht.

3. Verbindungen nach Anspruch 1 oder Anspruch 2 der allgemeinen Formel (I), in der X —NH— und $R_3$ eine Alkylgruppe oder eine Phenylalkylgruppe mit höchstens 8 Kohlenstoffatomen bedeuten.

4. Verbindungen nach einem der Ansprüche 1 bis 3 der allgemeinen Formel (I), in der R eine Methylengruppe darstellt.

5. N-[(R,S)-3-Thio-2-methyl-propionyl]-(R,S)-2-aza-3-carboxy-bicyclo[2.2.2]octan und dessen (S)-Isomere.

6. N-[(R,S)-N-(1-Ethoxycarbonyl-3-methyl-butyl]-(S)-alanyl]-(S)-2-aza-3-carboxy-bicyclo[2.2.2]octan, dessen (S)-Isomeres und deren Natriumsalz.

7. N-[(R,S)-N-(1-Ethoxycarbonyl-3-phenyl-propyl)-(S)-alanyl]-(S)-2-aza-3-carboxy-bicyclo[2.2.2]octan, dessen (S)-Isomeres, deren Natriumsalz und deren Trifluoracetat.

8. Pharmazeutische Zubereitung enthaltend als Wirkstoff mindestens eine Verbindung nach einem der Ansprüche 1 bis 7 sowie ein inertes, nichttoxisches, geeignetes Bindemittel oder Trägermaterial.

9. Verfahren zur Herstellung der Verbindungen nach Anspruch 1, dadurch gekennzeichnet, daß man eine Azabicyclooctancarbonsäure oder einen ihrer Ester der allgemeinen Formel (II)

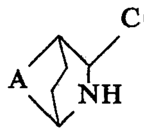

(II)

in der die Definition des Rests A die gleiche ist wie die für die Formel (I) des Anspruchs 1 und R' eine Hydroxylgruppe oder eine $C_1-C_4$-Alkoxygruppe bedeutet, der Einwirkung einer substituierten Carbonsäure der allgemeinen Formel (III)

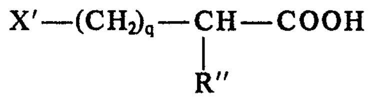

(III)

in der q die bezüglich der Formel (I) in Anspruch 1 angegebene Bedeutung besitzt und R'' eine $C_1-C_4$-Alkylgruppe oder eine geschützte $C_1-C_4$-Aminoalkylgruppe und X' eine durch übliche Acylreste geschützte SH- oder $NH_2$-Gruppe darstellen oder eines ihrer funktionellen Derivate aussetzt, zur Bildung einer Säure der allgemeinen Formel (IV)

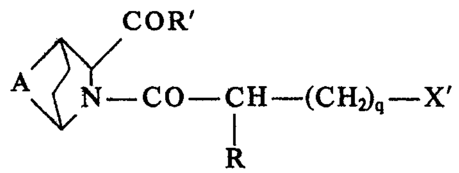

(IV)

in der die Substituenten die oben angegebenen Bedeutungen besitzen, welche den üblichen Maßnahmen zur Abspaltung der Schutzgruppen unterworfen wird und in dieser Weise die Verbindung der allgemeinen Formel (I) liefert, in der $R_1$ ein Wasserstoffatom darstellt, und gewünschtenfalls die Verbindung der Formel (I), in der $R_1$ ein Wasserstoffatom und X eine NH-Gruppe bedeuten, einer reduktiven Alkylierungsreaktion mit einer Verbindung der allgemeinen Formel (V)

$$R_2-CO-CO-R_3 \qquad (V)$$

in der $R_2$ und $R_3$ die bezüglich der Formel (I) angegebenen Bedeutungen besitzen, unterzieht zur Bildung einer Verbindung der Formel (I), in der X eine NH-Gruppe und $R_1$ eine Gruppe der Formel

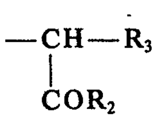

bedeuten.

**Patentanspruch für den Vertragsstaat AT**

Verfahren zur Herstellung von Verbindungen der allgemeinen Formel

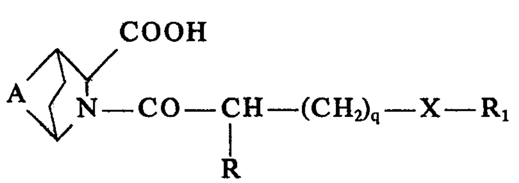

(I)

in der A eine Vinylengruppe oder eine Dimethylengruppe,

q 0 oder 1,
R eine $C_1-C_4$-Alkylgruppe, die eine Aminogruppe tragen kann,
X —S— und $R_1$ H oder X —NH— und $R_1$ ein Wasserstoffatom oder eine Gruppe der Formel

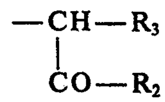

in der

$R_2$ eine Hydroxylgruppe oder eine $C_1-C_4$-Alkoxygruppe und
$R_3$ ein Wasserstoffatom, eine geradkettige oder verzweigte Alkylgruppe, eine Cycloalkylgruppe oder eine Phenylalkylgruppe mit höchstens 8 Kohlenstoffatomen darstellen, oder eine Gruppe der Formel

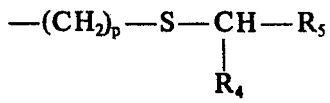

in der

$R_4$ ein Wasserstoffatom, eine $C_1-C_4$-Alkylgruppe oder eine $C_3-C_6$-Cycloalkylgruppe,
$R_5$ ein Wasserstoffatom, eine $C_3-C_6$-Cycloalkylgruppe oder $C_1-C_4$-Alkoxycarbonylgruppe und
p 1 oder 2 darstellen,

bedeuten, in Form des Racemats oder des optischen Isomeren, sowie ihrer Salze mit einer therapeutisch verträglichen anorganischen oder oganischen Base und der Additionssalze der Verbindungen der Formel (I), in der X eine NH-Gruppe darstellt, mit einer therapeutisch verträglichen anorganischen oder organischen Säure, dadurch gekennzeichnet, daß man eine Azabicyclooctancarbonsäure oder einen ihrer Ester der allgemeinen Formel (II)

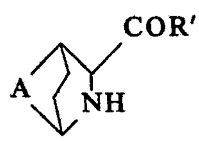

(II)

in der die Definition des Rests A die gleiche ist wie die für die Formel (I) und R′ eine Hydroxylgruppe oder eine $C_1-C_4$-Alkoxygruppe bedeutet, der Einwirkung einer substituierten Carbonsäure der allgemeinen Formel (III)

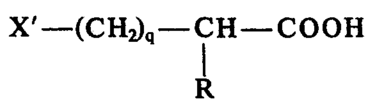

(III)

in der R und q die bezüglich der Formel (I) angegebenen Bedeutungen besitzen und X′ eine durch übliche Acylreste geschützte SH- oder $NH_2$-Gruppe darstellt oder eines ihrer funktionellen Derivate aussetzt, zur Bildung einer Säure der allgemeinen Formel (IV)

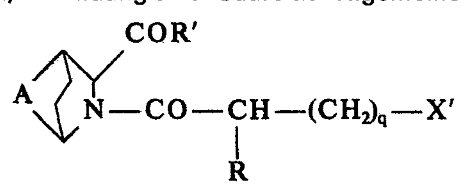

(IV)

in der die Substituenten die oben angegebenen Bedeutungen besitzen, welche den üblichen Maßnahmen zur Abspaltung der Schutzgruppen unterworfen wird und in dieser Weise die Verbindung der allgemeinen Formel (I) liefert, in der $R_1$ ein Wasserstoffatom darstellt, und gewünschtenfalls die Verbindung der Formel (I), in der $R_1$ ein Wasserstoffatom und X eine NH-Gruppe bedeuten, einer reduktiven Alkylierungsreaktion mit einer Verbindung der allgemeinen Formel (V)

$$R_2-CO-CO-R_3 \qquad (V)$$

in der $R_2$ und $R_3$ die bezüglich der Formel (I) angegebenen Bedeutungen besitzen, unterzieht zur

Bildung einer Verbindung der Formel (I), in der X eine NH-Gruppe und $R_1$ eine Gruppe der Formel

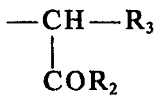

bedeuten.

**Claims for contracting states BE, CH, DE, FR, GB, IT, LI, LU, NL, SE**

1. Compounds corresponding to the general formula

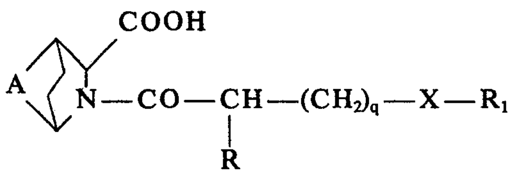

(I)

in which

A represents a vinylene or dimethylene radical,
q is 0 or 1,
R represents a $C_1$ to $C_4$-Alkyl radical that can have an amino group,
X represents —S— and $R_1$ represents H, or, alternatively,
X represents —NH— and $R_1$ represents a hydrogen atom or a radical of the formula

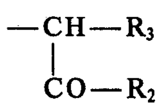

$R_2$ represents hydroxy or a $C_1$ to $C_4$-alkoxy grouping, and
$R_3$ represents a hydrogen atom, or a straight-chain or branched alkyl radical, a cycloalkylalkyl radical or a phenylalkyl radical each having not more than 8 carbon atoms in total, or a radical of the formula

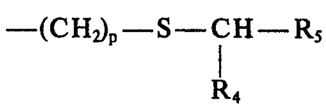

in which

$R_4$ is H, a $C_1$ to $C_4$-alkyl radical or a $C_3$ to $C_6$-cycloalkyl radical,
$R_5$ is H, a $C_3$ to $C_6$-cycloalkyl radical or a ($C_1$ to $C_4$-alkoxy)carbonyl radical, and
p is 1 or 2,

in racemic form or in the form of an optical isomer, and their salts obtained with a therapeutically compatible mineral or organic base and the addition salts of compounds of the formula (I) in which X is NH with a therapeutically compatible mineral or organic acid.

2. Compounds according to claim 1, corresponding to the formula (I) in which A represents a dimethylene group.

3. Compounds according to claim 1 or claim 2 corresponding to the formula (I) in which X represents NH, and $R_3$ represents an alkyl or phenylalkyl group having a maximum of 8 carbon atoms.

4. Compounds according to any one of claims 1 to 3 corresponding to the formula (I) in which R is a methyl group.

5. 2-[N-(3-thio-2-(RS)-methylpropionyl)aza]-3-(RS)-carboxybicyclo[2.2.2]octane and its S-isomers.

6. 2-{N-[N-(1-(RS)-ethoxycarbonyl-3-methylbutyl)—(S)-alanyl]aza}-3-(S)-carboxybicyclo[2.2.2]octane, its S-isomer and their sodium salts.

7. 2-{N-[N-(1-(RS)-ethoxycarbonyl-3-phenylpropyl)-(S)-alanyl]aza}-3-(S)-carboxybicyclo[2.2.2]octane, its S-isomer, their sodium salts and their trifluoroacetates.

8. Pharmaceutical composition containing as active ingredient at least one compound according to any one of claims 1 to 7 and a suitable, non-toxic, inert excipient or carrier.

9. Process for the preparation of the compounds according to claim 1, characterised in that an azabicyclo-octanecarboxylic acid, or an ester thereof, of the general formula (II)

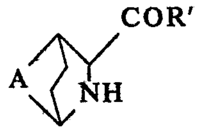

(II)

in which the definition of the substituent A is the same as in the formula (I) of claim 1 and R′ represents hydroxy or $C_1$ to $C_4$-alkoxy is subjected to the action of a substituted carboxylic acid of the general formula (III)

$$X'-(CH_2)_q-\underset{\displaystyle R''}{\underset{|}{CH}}-COOH \qquad (III)$$

in which q has the definition given for the formula (I) of claim 1, R″ is $C_1$ to $C_4$-alkyl or protected aminoalkyl (alkyl having 1 to 4 carbon atoms), and X′ represents SH or an $NH_2$ grouping protected by the customary acyl radicals, or a functional derivative thereof, to obtain an acid of the general formula (IV)

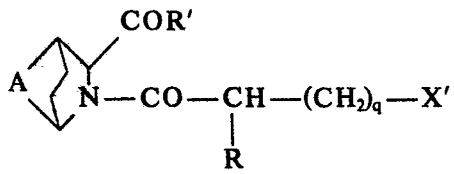

(IV)

in which the substituents have the meanings already mentioned above, which acid is subjected to customary deprotection processes and thus yields the compound of the general formula (I) in which $R_1$ is H, and then, if necessary, the compound of the formula (I) in which $R_1$ is H and X is NH is subjected to a reductive alkylation reaction using a compound of the general formula (V)

$$R_2-CO-CO-R_3 \qquad (V)$$

in which $R_2$ and $R_3$ have the meanings indicated in formula (I), to obtain a compound of the formula (I) in which X is NH and $R_1$ is

$$-\underset{\displaystyle COR_2}{\underset{|}{CH}}-R_3$$

**Claim for the contracting state At**

Process for the preparation of compounds corresponding to the general formula

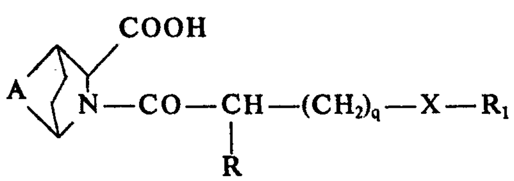

(I)

in which

A represents a vinylene or dimethylene radical,
q is 0 or 1,
R represents a $C_1$ to $C_4$-alkyl radical that can have an amino group,
X represents $-S-$ and $R_1$ represents H, or, alternatively,
X represents $-NH-$ and $R_1$ represents a hydrogen atom or a radical of the formula

$$-\underset{\displaystyle CO-R_2}{\underset{|}{CH}}-R_3$$

$R_2$ represents hydroxy or a $C_1$ to $C_4$-alkoxy grouping, and

$R_3$ represents a hydrogen atom, or a straight-chain or branched alkyl radical, a cycloalkylalkyl radical or a phenylalkyl radical each having not more than 8 carbon atoms in total, or a radical of the formula

$$-(CH_2)_p-S-\underset{\displaystyle R_4}{\underset{|}{CH}}-R_5$$

in which

$R_4$ is H, a $C_1$ to $C_4$-alkyl radical or a $C_3$ to $C_6$-cycloalkyl radical,

$R_5$ is H, a $C_3$ to $C_6$-cycloalkyl radical or a ($C_1$ to $C_4$-alkoxy)carbonyl radical, and

p is 1 or 2,

in racemic form or in the form of an optical isomer, and their salts obtained with a therapeutically compatible mineral or organic base and the addition salts of compounds of the formula (I) in which X is NH with a therapeutically compatible mineral or organic acid, process characterised in that an azabicyclo-octanecarboxylic acid, or an ester thereof, of the general formula (II)

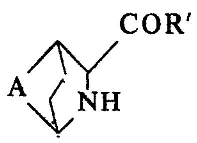

(II)

in which the definition of the substituent A is the same as in formula (I) and R′ represents hydroxy or $C_1$ to $C_4$-alkoxy is subjected to the action of a substituted carboxylic acid of the general formula (III)

$$X'-(CH_2)_q-\underset{\displaystyle R}{\underset{|}{CH}}-COOH \qquad \text{(III)}$$

in which R and q have the definitions given for formula (I) and X′ represents SH or an $NH_2$ grouping protected by the customary acyl radicals, or a functional derivative thereof, to obtain an acid of the general formula (IV)

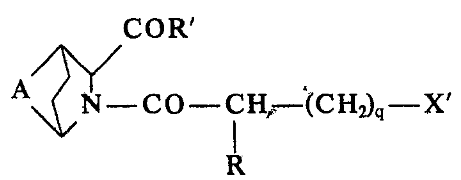

(IV)

in which the substituents have the definitions already mentioned above, which acid is subjected to customery deprotection processes and thus yields the compound of the general formula (I) in which $R_1$ is H, and then, if necessary, the compound of the formula (I) in which $R_1$ is H and X is NH is subjected to a reductive alkylation reaction using a compound of the general formula (V)

$$R_2-CO-CO-R_3$$

in which $R_2$ and $R_3$ have the meanings indicated in formula (I), to obtain a compound of the formula (I) in which X is NH and $R_1$ is

$$-\underset{\displaystyle COR_2}{\underset{|}{CH}}-R_3$$